# EUROPÄISCHE PATENTSCHRIFT

(11) **EP 2 036 496 B1**
(45) Veröffentlichungstag und Bekanntmachung des Hinweises auf die Patenterteilung: **09.08.2017**
(21) Anmeldenummer: 08170252.4
(22) Anmeldetag: 17.06.2005
(51) Int. Cl.: A61B 5/113, A61B 5/0408, A41D 13/12, A61B 5/00, A61B 5/0205, A61B 5/0444, A61B 5/0428

(54) **Kleidungsstück mit integrierter Sensorik**
Clothing with integrated sensors
Article d'habillement à technique sensorielle intégrée

(30) Priorität: 23.06.2004 DE 102004030261
(43) Veröffentlichungstag der Anmeldung: 18.03.2009
(62) Teilanmeldung aus: 05762685.5
(73) Patentinhaber: Deutsche Institute für Textil- und Faserforschung Denkendorf, 73770 Denkendorf (DE)
(72) Erfinder: Linti, Carsten, 70372 Stuttgart (DE); Planck, Heinrich, 72622 Nürtingen (DE); Horter, Hansjürgen, 72644 Oberboihingen (DE); Gutknecht, Ursula, 72805 Lichtenstein (DE)
(74) Vertreter: Rüger, Barthelt & Abel

(56) Entgegenhaltungen:
- WO-A-01/01855
- WO-A-02/40091
- WO-A-02/071935
- DE-T2- 69 314 225
- DE-U1- 20 210 134
- US-A- 4 729 377
- US-A- 4 960 118
- US-A- 5 295 490
- US-A1- 2002 124 295
- US-A1- 2004 073 104
- US-B1- 6 381 482
- CATRYSSE M ET AL: "Fabric sensors for the measurement of physiological parameters", TRANSDUCERS, SOLID-STATE SENSORS, ACTUATORS AND MICROSYSTEMS, 12TH INNATIONAL CONFERENCE ON, 2003, PISCATAWAY, NJ, USA,IEEE, Bd. 2, 9. Juni 2003 (2003-06-09), Seiten 1758-1761, XP010647504, ISBN: 0-7803-7731-1
- PARADISE R ET AL: "Knitted bioclothes for cardiopulmonary monitoring", PROCEEDINGS OF THE 25TH. ANNUAL INTERNATIONAL CONFERENCE OF THE IEEE ENGINEERING IN MEDICINE AND BIOLOGY SOCIETY. CANCUN, MEXICO, SEPT. 17, Bd. VOL. 4 OF 4. CONF. 25, 17. September 2003 (2003-09-17), Seiten 3720-3723, XP010691633, ISBN: 0-7803-7789-3
- WIJESIRIWARDANA R ET AL: "Resistive fibre-meshed transducers", WEARABLE COMPUTERS, 2003. PROCEEDINGS. SEVENTH IEEE INTERNATIONAL SYMPOSIUM ON 21-23 OCT. 2003, PISCATAWAY, NJ, USA,IEEE, 21. Oktober 2003 (2003-10-21), Seiten 200-209, XP010673810, ISBN: 0-7695-2034-0
- NEUMAN M R ET AL: "FABRICATING BIOMEDICAL SENSORS WITH THIN-FILM TECHNOLOGY", IEEE ENGINEERING IN MEDICINE AND BIOLOGY MAGAZINE, IEEE INC. NEW YORK, US, Bd. 13, Nr. 3, 1. Juni 1994 (1994-06-01), Seiten 409-419, XP000456214, ISSN: 0739-5175

## Beschreibung

Bei einer Reihe von Krankheiten/Situationen ist es zweckmäßig, zwecks Diagnose und Therapie den jeweiligen Menschen/Patienten kontinuierlich zu überwachen. Die Überwachung umfasst kardiale Funktionen der Atmung, dem Hautwiderstand, die Transpiration, die Körpertemperatur und dergleichen. Je nach Art der Erkrankung oder überwachten Situation ist ein unterschiedlicher Mix von Parametern zweckmäßig. Dabei soll die Messung kontinuierlich über einen langen Zeitraum erfolgen, und nicht etwa nur über wenige Minuten. Dies erfordert, dass die Sensoren, die am Körper angebracht werden, den Komfort bzw. die normale Bewegungsfreiheit nicht nennenswert beeinträchtigen.

Situationen, in denen eine Überwachung der Vitalparameter notwendig ist, könne in jeder Lebensphase auftreten. Beispielsweise müssen in medizinisch begründeten Fällen Atmungsaussetzer oder kardiale Fehler erkannt oder Rehabilitationen unterstützt werden (Altenpflege, Telemedizin u.ä.). In der Arbeitssicherheitssituationen ist eine Überwachung erforderlich um eine Überlastung oder unzulässige Gefährdung auszuschließen. In Fitness-, Sport- oder Wellnessanwendungen kann mittels einer Überwachung der Trainingserfolg protokolliert oder das Training unterstützt werden.

Besonders kompliziert bei der Überwachung sind Säuglinge und Kleinkinder, die sich durch eine ausgeprägte Motorik auszeichnen. In jedem Fall müssen die Sensoren ständig in Körperkontakt gehalten werden, um Fehlmessungen zu vermeiden. Andererseits darf von den Anschlüssen der Sensoren keinerlei Gefährdung für die überwachte Person bzw. die kleinen Patienten ausgehen.

Ein Kleidungsstück mit einem Sensor gemäß dem Oberbegriff des Patentanspruches 1 ist aus DE 693 14 225 T2 bekannt.

Ausgehend hiervon ist es deswegen Aufgabe der Erfindung eine Anordnung zu schaffen, mit der ein Sensor lagerichtig an einem menschlichen Körper fixiert werden kann.

Diese Aufgabe wird erfindungsgemäß durch das Kleidungsstück mit den Merkmalen des Anspruches 1 gelöst.

Gemäß der Erfindung wird ein Kleidungsstück verwendet, dessen Material, zumindest abschnittsweise, zumindest in einer Richtung dehnbar ist. Aufgrund der Dehnbarkeit wird die Bewegung relativ wenig eingeschränkt und andererseits sorgt die Dehnbarkeit dafür, dass die Sensoren genügend im Körperkontakt bleiben. Der Schnitt ist so gewählt, dass das Kleidungsstück im angelegten Zustand am Körper lagerichtig fixiert bleibt.

In dem Kleidungsstück befindet sich wenigstens ein Sensor zum Erfassen einer Vitalfunktion, wie Hautwiderstand, Transpiration, Atmung, Puls, Herzströme, Körpertemperatur und ähnlichem. Die Sensoren geben ein elektrisches Signal ab, das entweder ein umgewandeltes, eingespeistes elektrisches Signal ist, oder sie dienen als Schnittstelle zum Ableiten von Körperströmen in eine Messapparatur.

Hierzu ist ferner ein Verbindungskabel vorgesehen, das aus dem Kleidungsstück herausführt und in dem Kleidungsstück befestigt ist.

Um den Sensor in einem möglichst engen Kontakt zum Körper zu halten, ist der Sensor in oder an einem, zumindest abschnittsweise, dehnbaren, vorzugsweise elastisch dehnbaren Gürtel angebracht. Bei dem Gürtel kann es sich um einen flachliegenden Schlauch handeln. Der Schlauch kann als Rundgestrick oder Rundgewirke erzeugt sein. Dies hat den Vorteil, dass keine Nähte auftreten, die den Tragekomfort beeinträchtigen, indem sie beispielsweise die Haut wund scheuern oder die Dehnbarkeit behindern.

Außerdem gestattet der schlauchförmige Gürtel eine schützende Unterbringung des Sensors, soweit kein unmittelbarer Hautkontakt erforderlich ist.

Besonders zweckmäßig ist es, wenn es sich bei dem Kleidungsstück um einen so genannten Body handelt, der Thorax und Abdomen umhüllt und mit einem Halsausschnitt und zwei Armausschnitten sowie Beinausschnitten versehen ist.

Um das Anziehen eines solchen Bodys an einem Säugling oder einem Kleinkind zu erleichtern, ist der Body in Längsrichtung zu öffnen.

Außerdem ist es vorteilhaft, wenn der Body einen angeschnittenen Schrittspickel enthält, der durch den Schritt geführt wird.

Insbesondere, wiederum für Säuglinge und Kleinkinder, ist es vorteilhaft, wenn der Body mit Ärmeln versehen ist, damit er nicht nur als Träger für die Sensoren dient, sondern ein vollständiges Kleidungsstück bildet, das den Körper gegen Auskühlung schützt.

Es ist jedoch auch möglich, das Kleidungsstück als Weste, nach Art eines T-Shirts oder eines Unterhemds mit Trägern auszuführen.

Wenn das Material des Kleidungsstücks in allen Richtungen elastisch dehnbar ist, kann sich das Kleidungsstück sehr gut an die Gestalt des Trägers anpassen ohne nennenswerte Zwänge auszuüben oder Falten zu bilden, wenn sich der Träger bewegt.

In sehr günstiger Weise werden diese Forderungen erfüllt, wenn es sich bei dem Material um ein textiles Flächengebilde handelt. Das textile Flächengebilde ist vorzugsweise eine Maschenware, die von sich aus die notwendige Elastizität mitbringt. Das Material für die Maschenware kann normale Baumwolle sein, die, gegebenenfalls in einem geringen Umfang, beispielsweise weniger als 5%, Elastanfäden enthält. Die Baumwollfasern erhöhen erheblich den Tragekomfort. Viskose-, Kunst- oder Mikrofasern sind einsetzbar und können die Funktion des Textils unter Umständen erheblich erweitern, indem sie klimatisierend wirken, Hauterkrankungen wie Neurodermitis lindern oder ähnliches.

Als Sensoren kann es sich um Sensoren handeln, die bei Streckung ihren Widerstandswert verändern. Vorzugsweise beträgt der spezifischen Widerstand des Sensors 25 Ohmcm. bzw. der Wert kann in einem Bereich zwischen 5 Ohmcm und 30 kOhmcm liegen, wobei jeder dazwischen liegende Bereich als Wert eines neu zu definierenden Bereiches mit beansprucht ist.

Mit einer solchen Art von dehnungsabhängigem Sensor wird das eingespeiste elektrische Signal modifiziert, da der durch den Sensor fließende Strom je nach Widerstandswert vergrößert oder verkleinert wird. Im Falle eines Speisens des Sensors mit konstantem Strom ändert sich nicht der Strom, sondern der Spannungsabfall, so dass dieser als Signal zu verstehen ist.

Der dehnungsabhängige Sensor kann erreicht werden, indem ein an sich nicht leitendes elastomeres Grundmaterial verwendet wird, in dem leitfähige Partikel eingebettet sind. Bei den leitfähigen Partikeln kann es sich um Kohlepartikel handeln oder um leitfähige Metallpartikel, d.h. Metallpartikel, die nicht an der Oberfläche durch Oxidation eine nichtleitende Haut bekommen haben oder innerhalb sehr kurzer Zeit bekommen, selbst wenn sie in dem Elastomer eingebettet sind.

Eine andere Form eines dehnungsabhängigen Sensors basiert auf einem Hydrogel.

Das Elastomer ist zweckmäßigerweise ein hautverträgliches Elastomer, das, zumindest überwiegend, nicht allergen ist. Diese Bedingung ist nur dann von besonderer Bedeutung, wenn es sich um einen Sensor handelt, der unmittelbar auf der Haut getragen wird, wie Sensoren/Elektroden zum Ableiten von Herzströmen oder zum Messen des Hautwiderstands.

Zweckmäßigerweise ist das Elastomer stärker dehnbar, als das Substrat, auf dem sich der Sensor befindet. Auf diese Weise wird vermieden, dass die Dehnbarkeit des Sensors die Dehnbarkeit des Substrates, in diesem Fall des Kleidungsstücks oder eines Teils des Kleidungsstücks, begrenzt. Günstige Materialien als Substrat sind Fluorelastomere, Polyurethane oder Silikon.

Je nach Anwendungsfall kann es von Vorteil sein, wenn der Sensor auf zumindest einer Seite mit einer Isolierschicht versehen ist, die zweckmäßigerweise dehnbar ist. Dies kann beispielsweise eine Zwischenschicht sein, zwischen der eigentlichen aktiven Fläche und dem Substrat in Gestalt des Kleidungsstücks, oder aber eine Isolierschicht zwischen dem aktiven Teil des Sensors und der Haut im Falle von Dehnungssensoren.

Damit Feuchtigkeit das Messsignal im Falle von Dehnungssensoren nicht beeinflusst, ist bei diesen die aktive Schicht zweckmäßigerweise allseitig von Isolierschichten umgeben.

In jedem Falle können die Isolierschichten aus demselben Grundmaterial bestehen wie die aktiven Schichten.

Zur Ableitung der elektrischen Signale ist der Sensor über wenigstens einen Anschlussdraht kontaktiert. Im Falle von Dehnungssensoren sind selbstverständlich zwei Anschlussdrähte erforderlich.

Gute elektrische Signale werden im Falle von Dehnungssensoren erhalten, wenn der Dehnungssensor bahnförmig gestaltet ist, d.h. die Quererstreckung ist klein gegenüber der Längserstreckung. Die Empfindlichkeit lässt sich noch weiter steigern, wenn die Bahn des Sensors wenigstens einmal U-förmig verläuft, wobei unter mehrfach U-förmig auch einer Z-förmiger Verlauf verstanden werden soll. Dadurch lässt sich die Längserstreckung des Dehnungssensors verkleinern, verglichen mit einem Dehnungssensor, der nur eine Bahn in Längsrichtung aufweist und die gleiche Empfindlichkeit zeigt.

Im Falle von Sensoren zum Ableiten von Herzströmen, die im Wesentlichen nur als Kontaktflächen dienen, ist die flächige Gestalt auf der dem Körper zugewandten Seite vorteilhaft. Die Form kann rund oder eckig sein, je nach den erfordernissen. Es soll eine große Kontaktfläche erreicht werden, ohne dass Dehnungen auftreten können, die den Widerstandswert des Sensors nennenswert beeinflussen.

Der Sensor muss so flexibel und drapierbar sein, dass er sich der Körperoberfläche gut anpasst. Die Oberfläche kann glatt oder strukturiert sein. Die Struktur kann sich aus Pyramiden oder Tetraedern zusammensetzen, damit der Hautschweiß besser abgeleitet werden kann. Die Spitzen erhöhen den lokalen Anpressdruck auf der Haut und erzeugen somit lokal einen besseren Hautkontakt. Allerding darf die Struktur nicht so ausgeprägt sein, als dass sie zu einer bleibenden Schädigung der Haut oder einem unangenehmen Tragegefühl führt.

Der Sensor sollte aus einem Material bestehen, das gegen Körperschweiß und Körperausdünstungen unempfindlich ist. Diese Unempfindlichkeit sollte, zumindest für die Oberflächenschichten gegeben sein, soweit diese den Kern hinreichend schützen können.

Damit der Sensor die Reinigung des Kleidungsstückes und/oder die Desinfektion und/oder Sterilisation nicht beeinträchtigen, sollte der Sensor aus Materialien bestehen, die unter normalen Bedingungen waschbeständig sind um eine einfache Pflege zu ermöglichen, heißwasserfest sind um eine weitgehende Desinfektion zu ermöglichen oder die es sogar in dem Maße warmfest sind, dass sie eine Sterilisation im Autoklaven überstehen.

Der Gürtel besteht zweckmäßigerweise aus einer Maschenware, die eine Dehnbarkeit in Längsrichtung des Gürtels ermöglicht. Hierdurch wirkt der Gürtel nicht beengend. Weder die Brust- noch die Bauchatmung des zu überwachenden Patienten/Person wird beeinträchtigt. Der Gürtel verläuft in dem Kleidungsstück quer zur Längsachse des Körpers, beispielsweise, wenn die Atmung überwacht wird. Es sind zwei Gürtel in dem Kleidungsstück vorhanden, wodurch sowohl die Brust- als auch die Bauchatmung kontrolliert werden kann.

Die zwei Gürtel und das Verbindungskabel bilden eine F-förmige Konfiguration. Dazu sind zwei alternative Möglichkeiten vorgesehen, die einfach und effizient herstellbar sind: das Verbindungskabel ist ein zweilagiges Gebilde mit zwei Lagen, die zur Bildung der F-förmigen Konfiguration voneinander getrennt und umgeklappt sind; oder das Verbindungskabel ist ein Bandkabel, das zur Bildung der F-förmigen Konfiguration der Länge nach getrennt und umgeschlagen ist.

Als weiteres Material für den Gürtel kommt auch ein dehnbares Gewebe oder Vlies in Frage. In dem Vlies oder dem Gewebe können dehnbare Fäden eingelegt, eingenäht oder aufgestickt sein.

Der Gürtel kann auch in einem Herstellungsschritt zusammen mit dem Textil hergestellt werden (spezielle Strick-, Wirk- oder Webtechniken sind hierfür geeignet, nämlich sogenannte Fully Fashioned), wobei Bereiche des Textils, die Gürtelbereiche mit angepassten Dehnungseigenschaften ausgebildet werden können. Die Dehnung im Rücken kann kleiner sein während die Dehnung Brust- und Bauchbereich stärker ist.

Speziell die Flachstricktechnik oder auch die Schaft- und Jaquardwebtechnik ermöglichen es, Funktionen wie Steifigkeitsänderungen und ortsabhängig unterschiedliche Einbindungen von verschiedenen Materialien in die Fläche zu integrieren. Beim Stricken sind Flottungen durch Unter- oder Überlegungen möglich. Beim Weben ermöglichen dies Bindungsformen, wie die Leinwand, die Köperbindung oder die freie Bindung. Unter "fully fashioned" versteht der Fachmann eine Flachstricktechnologie, die das Herstellen eines Kleidungsstückes in einem Arbeitsgang ohne anschließenden Näharbeiten gestattet. Durch Umhängen von Maschen und andere Techniken, können bei der "fully fashioned"-Technologie zusätzlich zur Gesamtformgebung auch andere über die Bindungs- und Musterungstechnik hinausgehende Formgebungsmöglichkeiten erreicht werden. So kann der Gürtel gleich bei der Herstellung des Kleidungsstücks ohne zusätzliche Zuschneid- und Näharbeiten integriert werden. Durch Wahl der Maschenbildung, Maschenweite und des Garns können in weiten Bereichen unterschiedliche Eigenschaften erzeugt werden.

Um ein Verschieben des Gürtels in dem Kleidungsstück zu vermeiden, ist der Gürtel, zumindest abschnittsweise, mit dem Kleidungsstück vernäht. Andere Abschnitte können frei sein, so dass der Gürtel unabhängig von dem Sitz des Kleidungsstückes hinreichend straff gezogen werden kann.

Zum Schließen des Gürtels ist an diesem ein Druckknopf oder ein Klettband vorgesehen.

Der Schutz der Anschlussleitungen wird verbessert, wenn der Gürtel in einen schlauchförmigen Bereich des Kleidungsstücks einmündet, durch den das Anschlusskabel hindurchgeführt ist.

Zum elektrischen Verbinden der Sensoren mit der Auswerteelektronik können Einzeladern verwendet werden, von denen jede für sich isoliert ist. Diese Einzeladern können in einem Gewebe eingebunden werden, und zwar als dessen Kettfäden. Dadurch wird ein robustes Flachbandkabel erzielt, das sehr flexibel ist, und das sich wegen der entsprechenden Breite kaum verdrehen kann. Gleichzeitig schützen die im Wesentlichen undehnbaren, nicht leitenden Kettfäden die empfindlichen Drähte gegen Überdehnung, Abreißen oder Bruch durch zu enge Biegeradien. Die isolierten Einzeladern könne auch als Stehfäden in Wirkware eingebracht werden.

Die Kontaktierung mit den textilintegrierten oder auf das Textil aufgebrachten Sensoren kann mit konfektionstechnischen Methoden erfolgen. Dazu können Leiterschlaufen zur Zugentlastung, meanderförmige Schlaufen zur Erhöhung der Dehnbarkeit sowie abisolierte Enden mit dem Textil vernäht, aufgestickt, aufgeklebt oder durch bekannte Techniken verschweißt werden. Auf die leitfähigen abisolierten Kabelenden können die Sensoren elektrisch leitfähig geklebt, gelötet, ggf. gestrickt, genäht, geschweißt oder durch Beschichtung aufgebracht werden. Einige dieser Arbeitsschritte können auch gemeinsam ausgeführt werden, um die leitfähige Kontaktierung und die evtl. erforderliche Isolierung in einem Arbeitsgang auszuführen.

In den Zeichnungen sind Ausführungsbeispiele des Gegenstandes der Erfindung dargestellt. Es zeigen:
- Fig. 1: einen Body, insbesondere für einen Säugling, in einer ausgebreiteten Darstellung mit Blick auf die Innenseite;
- Fig. 2: den prinzipiellen Aufbau eines Dehnungssensors in der Draufsicht;
- Fig. 3: den Dehnungssensor in einem Querschnitt;

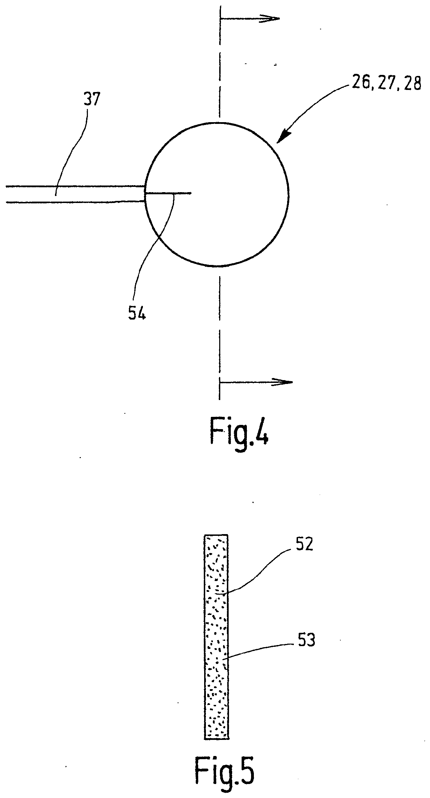

- Fig. 4: einen Ausschnitt aus dem Flachbandkabel zum Anschluss des Sensors;
- Fig. 5: einen Sensor bzw. eine Elektrode zum Ableiten von Herzströmen oder zum Messen des Hautwiderstands, in einer vergrößerten Schnittdarstellung;
- Fig. 6: ein weiteres Ausführungsbeispiel eines Bodies, bei dem die elektrischen Leitungen unmittelbar in das Textil des Bodies eingearbeitet sind,
- Fig. 7 und 8: Hosen mit integrierten Sensoren,
- Fig. 9-Fig. 11: unterschiedliche Ausführungen von Bandkabeln in einer Draufsicht,
- Fig. 12-Fig. 16: unterschiedliche Arten der Befestigung eines Drahtes auf einer textilen Struktur,
- Fig. 17: die Oberflächenstruktur eines Sensors, in einer Draufsicht und
- Fig. 18: die Oberflächenstruktur nach Fig. 17, in einer Seitenansicht.

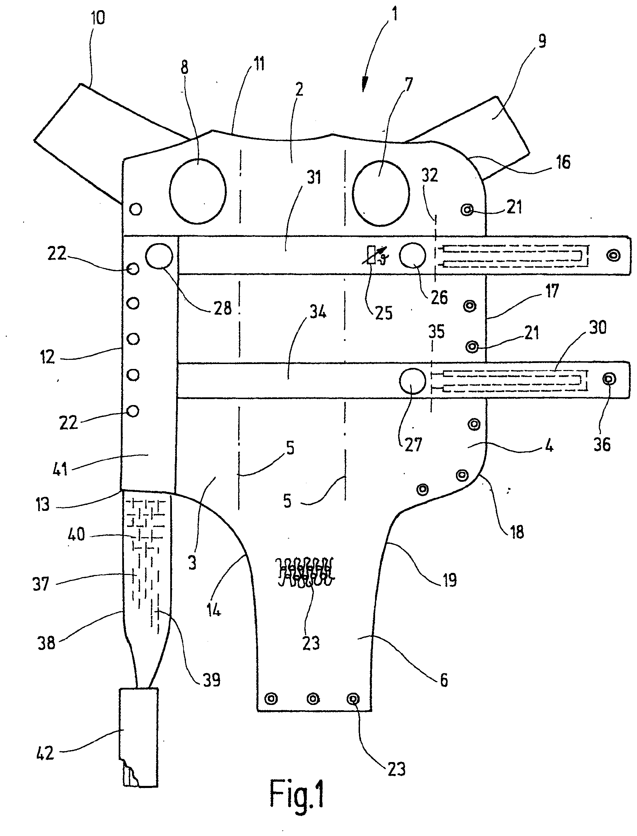

Fig. 1 zeigt einen Body 1 für Säuglinge und Kleinkinder. Der Body 1 ist in aufgeklappter Stellung dargestellt mit Blick auf die Innenseite. An dem Body ist ein Rückenteil 2 zu erkennen, das einstückig in ein rechtes Vorderteil 3 sowie ein linkes Vorderteil 4 übergeht. Die beiden Vorderteile 3 und 4 sind gedanklich durch strichpunktierte Linien 5 von dem Rückenteil 2 abgetrennt. In der praktischen Ausführung sind die beiden Vorderteile 3 und 4 ohne Seitennaht angeschitten. Die Begriffe "Vorderteil" und "Rückenteil" werden hier in der Weise verwendet, wie dies in der textilen Konfektion üblich ist.

Am unteren Ende des Rückenteils 2 geht eine angeschnittene Klappe oder Schrittspickel 6 aus, der im Tragezustand durch den Schritt hindurch führt.

Am oberen Ende der beiden Vorderteile 3 und 4 befinden sich Armausschnitte 7 und 8, in die von den Armausschnitten 7 und 8 ausgehende Ärmel 9 und 10 angenäht sind.

Eine obere Kante 11 bildet im Tragezustand einen Halsausschnitt.

Das rechte Vorderteil 5 ist seitlich von einer geraden Kante 12 begrenzt, die an der Kante 11 für den Halsausschnitt beginnt und etwa bei 13, und somit auf einer Höhe, die den Übergang zwischen dem Rückenteil 2 in die Schrittspickel 6 kennzeichnet, in die Schrittspickel 6 mit einer gekrümmten Schnittkante 14 übergeht. Das linke Vorderteil 4 geht mit einer abgerundeten Kante 16 in eine gerade nach unten verlaufende Kante 17 über, die wiederum auf Höhe der Ecke 13 in einem abgerundeten Abschnitt 18 in den bogenförmig verlaufende Kante 19 übergeht, die gleichzeitig auch die seitliche Begrenzung der Schrittspickel 6 darstellt.

Die Quererstreckung des Vorderteils 4 ist größer als die Quererstreckung des Vorderteils 3, so dass im Tragezustand das Vorderteil 4 klappenartig auf der vom Körper abgewandten Seite das Vorderteil 3 überdecken kann.

Zum Fixieren des Bodys 1 im geschlossenen Zustand sind längs der Kante 17 bzw. 18 Druckknopfoberteile 21 vorhanden. Diese Druckknopfoberteile 21 korrespondieren mit Druckknopfunterteilen, die längs der Schnittkante 12 angeordnet sind. Von diesen Druckknopfunterteilen sind deren Nietringe 22 zu erkennen, die verwendet werden, um die c mit dem Body 1 zu vernieten.

Weitere Druckknopfoberteile 21 sind am unteren freien Ende des Schrittspickels 6 vorhanden. Sie korrespondieren mit Druckknopfoberteile, die an der Außenseite der beiden Vorderteile 3 und 4 angenäht sind und deswegen in der Figur nicht zu erkennen sind.

Anstelle der gezeigten Druckknöpfe zum Schließen des Textils können auch Knöpf, Häkchen oder Tentakelhaftverschlüsse vorgesehen werden.

Der insoweit beschriebene Body 1 dient als Träger für Sensoren, um Vitalfunktionen des Trägers überwachen zu können. Die Sensoren umfassen einen Temperatursensor 24, insgesamt 3 Elektroden 25, 26 und 27, um zweikanalig die Herzströme ableiten zu können, sowie zwei gestrichelt angedeutete Dehnungsmessstreifen 28 und 29 zur Erfassung der Brust- und der Bauchatmung. Weitere Sensoren in Form von Elektroden könne vorhanden sein um den Hautwiderstand zu messen oder die Transpiration.

Das Grundmaterial für den Body 1, einschließlich der Arme 9 und 10, besteht aus einer Maschenware, wie dies bei 23 angedeutet ist. Die Maschenware kann eine Trikotware, ein Gewirke oder ein Gestrick sein. Der Vorteil der Maschenware besteht darin, dass das textile Flächengebilde in beiden Achsrichtungen dehnbar ist und eine gewisse Rücksprungkraft aufweist. Aufgrund dieser Eigenschaft wird ein strammer Sitz gewährleistet, der auch bei Bewegungen nicht zum bilden von Falten neigt.

Das Anlegen am Körper kann noch verbessert werden, wenn gegebenenfalls noch ein Elastomerfaden, beispielsweise Elastan, in geringem Umfang mit eingestrickt ist.

Die Art des Einstrickens von Elastanfäden ist aus dem Stand der Technik bekannt und braucht deswegen hier nicht weiter erläutert zu werden.

Der Dehnungsmessstreifen 29 befindet sich in einem Gürtel 31, der als Gestrickschlauch ausgeführt ist. Die Maschenstäbchen liegen in Längsrichtung des Gürtels 31. Der Gürtel 31 ist etwa an einer gestrichelt angedeuteten Stelle 32 mit dem Body 1 vernäht. Der Gürtel 31 beginnt in der Nähe der Schnittkante 12 und reicht, wie gezeigt, über die Schnittkante 17 hinaus. Er liegt rechtwinklig zur Längsachse des menschlichen Körpers, wenn der Body 1 getragen wird. Außerdem ist er so bemessen, dass er im Tragezustand des Bodys aus dem Überlappungsspalt zwischen den beiden Vorderteilen 3 und 4 herausführt. Zum Befestigen des freien Endes des Gürtels 31 ist ein weiterer Druckknopf 33 vorgesehen, mit dem an der Außenseite des Vorderteils 3 bzw. des Rückenteils 2 befindliche Druckknopfpfannen korrespondieren. Sie sind aus Darstellungsgründen in Fig. 1 nicht zu erkennen. Im Wesentlichen liegen sie verdeckt durch den Gürtel 31.

Da der Gürtel 31, wie erwähnt, als Schlauch ausgeführt ist, kann sich der Dehnungsmessstreifen 29 geschützt im Inneren befinden. Eine mechanische Beschädigung ist weitgehend ausgeschlossen. Außerdem sind Hautirritationen, die von dem Dehnungsmessstreifen und dessen Kanten ausgehen könnten, ebenfalls vermieden, da sich zwischen der Haut des Trägers und dem Dehnungsmessstreifen 29 eine Lage Stoff befindet. Das Material für diesen Stoff kann dasselbe Material sein, wie es für den Hauptteil des Bodys 1 verwendet wird, nämlich im Wesentlichen Baumwolle oder jeder auf Kunstfaser basierende hautverträgliche Stoff, der einen sehr hohen Tragekomfort gewährleistet, da insbesondere auch Feuchtigkeit gut aufgenommen werden soll.

In der Nähe des Armausschnittes 7 befindet sich auf dem Gürtel 31, wie angedeutet, die Elektrode 26. Sie ist so platziert, dass beim Tragen des Bodies 1 die Elektrode an der Stelle am Körper anliegt, wie dies im Bereich der Elektrokardiografie bekannt ist. Die zweite Elektrode 28 befindet sich ebenfalls in Verlängerung des Gürtels 31 auf dergleichen Körperhöhe.

Ein weiterer Gürtel 34 verläuft quer zu dem Rückenteil 2 auf einer Höhe, die im Tragezustand etwa knapp über dem Bauchnabel entspricht. Der Aufbau des Gürtels 36 ist derselbe, wie der Aufbau des Gürtels 31 und er ist auch in ähnlicher Weise befestigt. Bis zu einer Stelle 35 ist der schlauchförmige Gürtel 34 fest mit dem rechten Vorderteil 3, dem Rückenteil 2 und dem linken Vorderteil 4 vernäht. Der anschließende Abschnitt bildet eine freie Schrittspickel, die den Dehnungsmessstreifen 30 enthält. Das freie Ende des Gürtels 34 ist mit einem Druckknopf 36 versehen, um den Gürtel unter Spannung am Körper des Trägers anliegend zu halten.

Auch der Gürtel 34 trägt eine Elektrode zur Ableitung von Herzströmen in Gestalt der Elektrode 27. Ihre Lage entspricht der Lage, die zur zweikanaligen Herzstromableitung notwendig ist.

Zum Ableiten der elektrischen Signale aus den Elektroden 26, 27, 28, dem Thermistor in Gestalt eines NTC-Widerstandes 24 sowie den beiden Dehnungsmessstreifen 29, 30 werden extrem feine isolierte Drähte verwendet, wie sie bei 37 gestrichelt angedeutet sind. Diese Drähte sind wegen ihrer Feinheit extrem empfindlich gegen Beschädigung. Um sie mechanisch zu schützen, sind sie Bestandteil eines Gewebebandes 38. Das Gewebeband 38 ist als Band mit geschlossenen Kanten gewebt, die nicht ausfransen können. In diesem Band bilden die isolierten Drähte 37 parallel nebeneinander verlaufende Kettfäden. Rechts und links dieser mittig verlaufenden elektrischen Leitungen sind Kettfäden 39 eingewebt, die aus Baumwolle oder Kunstfaser bestehen und weitgehend undehnbar sind.

Auch die Schussfäden 40 des Bandes 38 bestehen aus undehnbarer Baumwolle, Kunst- oder Mischfasern.

Das so erhaltene Bandkabel verläuft, abgedeckt durch eine aufgenähte Klappe 41, neben der Schnittkante 12. Auf der Höhe des Gürtels 34 zweigt rechtwinklig ein erster Abschnitt ab, der in dem Gürtel 34 hinein verläuft und dort entsprechend kontaktiert ist, ein weiterer Teil des bandförmigen Kabels 38 knickt etwa unterhalb der Elektrode 28 um, um die in dem Gürtel 31 enthaltenen Sensoren einschließlich der Elektrode 28 zu kontaktieren.

Das untere freie Ende des bandförmigen Kabels ist mit einem Stecker 42 versehen, um die Sensoren mit einer Auswerteelektronik elektrisch zu verbinden.

Aufgrund der speziellen Anordnung des bandförmigen Kabels 38, verläuft es im Tragezustand über die Mitte des Körpers in Richtung auf die Beine, wodurch sich eine möglichst geringe Behinderung ergibt und auch das Risiko klein gehalten wird, dass durch die Bewegungen des Trägers, insbesondere eines Säuglings, das Kabel abgerissen wird. Es kann in dem Beinausschnitt herausgeführt werden und behindert den Säugling nicht bei seiner natürlichen Bewegung auch dann nicht, wenn das Kind etwas größer ist und sich im Bett dreht. Eine Strangulationsgefahr ist ausgeschlossen

Gleichzeitig sorgt der Thorax und Abdomen vollständig einhüllende Body 1 dafür, dass die diversen Sensoren an der richtigen Stelle am Körper platziert bleiben. Sie können weder in Umfangsrichtung noch in Längsrichtung weg wandern. Die Vorspannung sorgt auch für den notwendigen Kontaktdruck, damit die elektrische Verbindung zwischen den Elektroden 26, 27, 28 und der Hautoberfläche bestehen bleibt. Das stramme Anliegen der Gürtel 31 und 34 führt dazu, dass die Ausdehnung in Folge von Brust- und Bauchatmung auch die Dehnungsmessstreifen 29 und 30 übertragen werden. Hierdurch ist ein Monitoring der Atmung des Trägers gewährleistet.

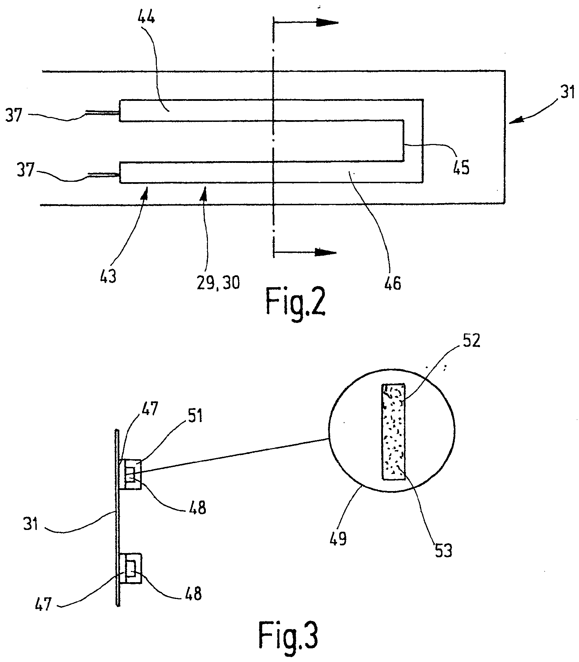

Der Dehnungsmessstreifen 29, 30 ist in Figur 2 im einzelnen gezeigt. Fig. 2 lässt den aufgeschnittenen schlauchförmigen Gürtel 31 erkennen, wobei auf der dem Träger oder Körper zugewandten Seite des flachliegenden Schlauches eine U-förmig verlaufende Bahn 43 aufgebracht ist. Die Bahn läuft mit einem ersten Schenkel 44 parallel zur Längserstreckung des Gürtels 31. Am Ende geht sie, entsprechend dem freien Ende des Gürtels 31, in einen Rückenabschnitt 45 über, der schließlich in einen Schenkel 46 einmündet, der parallel zu dem Schenkel 44 verläuft. An den freien Enden der beiden Schenkel sind die entsprechenden elektrischen Leitungen 37 angeschlossen.

Der Aufbau des Dehnungsmessstreifens 29 bzw. 30 ergibt sich aus der Schnittdarstellung nach Fig. 3. Hieraus ist zu erkennen, dass auf der Innenseite des Gürtels 31 zunächst eine isolierende Schicht 47 aufgebracht ist. Die isolierende Schicht 47 folgt dem Verlauf der Bahnen 44, 45, 46. Die isolierende Schicht 47 ist isolierend im elektrischen Sinne, d.h. sie ist extrem hochohmig.

Mittig auf der isolierenden Schicht 47 ist eine elektrisch leitende Schicht 48 aufgebracht. Die elektrisch leitende Schicht 48 ist schmäler, als es der isolierenden Schicht 47 entspricht. Sie setzt sich über die gesamte Länge der Bahnen 44, 45, 46 ununterbrochen fort.

Der innere Aufbau ist bei 49 vergrößert dargestellt.

Die elektrisch leitende Schicht 48 wird schließlich von einer weiteren isolierenden Schicht 51 überdeckt, wie dies die Schnittdarstellung erkennen lässt. Auf diese Weise ist die elektrisch leitende Schicht 48 allseitig umhüllt und lediglich an den Stirnenden der Bahnen 44 und 46 über die Leiter 37 elektrisch kontaktiert.

Das Material für die Schichten 47, 48, 51 ist ein Elastomer, das hautverträglich ist und zweckmäßigerweise auch nicht allergen. Geeignete Materialien sind Polyurethan, Silikon und Fluorelastomere. Außerdem haben diese Elastomere die Eigenschaft sehr dehnbar zu sein und die Dehnfähigkeit des Gürtels 31, der als Substrat für die Dehnungsmessstreifen 29, 30 dient, nicht zu behindern.

Die verwendenten Elastomere haben eine höhere Dehnfähigkeit als das textile Substrat, auf dem sie befestigt sind. Das textile Substrat schützt die elastische Struktur vor Überdehnung. Die Elastomere zeichnen sich z.B. im Falle von Silikon durch eine sehr geringe Steifigkeit und eine geringe Shore A-Härte von weniger als 20 aus. Bei geringen Schichtdicken von weniger als 1 mm wird durch das Elastomer die Dehnung des textilen Substrates unwesentlich behindert.

Außerdem sollte diese Elastomere, je nach Anwendungsbereich, zumindest warmwasserfest sein, damit der Body waschbar ist. Bei höheren Anforderungen an die Keimfreiheit kann auch eine Heißwasserfestigkeit erforderlich sein, um den Body 1 desinfizieren zu können. Gegebenenfalls könnte sogar eine Sterilisation im Autoklaven in Frage kommen, was die Anforderungen an die Temperatur- und die Dampffestigkeit der Elastomere weiter erhöht. Dasselbe gilt natürlich auch für die Isolation der Anschlussdrähte 37.

Da die genannten Elastomere im Wesentlichen elektrische Nichtleiter sind, kann die Leitfähigkeit der mittleren leitenden Schicht 48 nur erhalten werden, indem in entsprechender Menge leitende Partikel wie Kohlepartikel 52 eingebettet werden. Die Kohlepartikel werden in einem Mengenanteil eingebettet, bis ein spezifischer Widerstand von ca. 25 Ohm cm zustande kommt. Zweckmäßigerweise bewegt sich der spezifische Widerstand in einem Bereich zwischen 2 Ohm cm und 1 kOhm cm.

Aufgrund der in dem elastomer eingebetteten elektrisch leitenden Partikeln, ändert sich der spezifische Widerstand der elektrisch leitenden Widerstandsschicht 48 abhängig von der Dehnung. Da der Dehnungsmessstreifen 29 bzw. 30 U-förmig verläuft, wird ein höheres Nutzsignal erzeugt, weil zwei in Längsrichtung parallel zueinander verlaufende Bahnen gleichzeitig mit einer Dehnung beaufschlagt werden. Das Nutzsignal ist größer, als wenn nur eine Bahn verwendet würde. Eine noch größere Empfindlichkeit wird erreicht, wenn mehr als zwei Bahnen zueinander parallel verlaufen, soweit dies die Platzverhältnisse zulassen. Die Kontaktierung erfolgt zweckmäßigerweise, indem in das noch nicht ausgehärtete Elastomer der Widerstandsschicht 48 die abisolierten Enden der Anschlussdrähte 37 eingebettet werden. Sodann wird anschließend die isolierende Elastomerschicht 51 aufgebracht.

Anstelle von Kohlepartikeln können noch entsprechende Metallpartikel verwendet werden. Dabei ist darauf zu achten, dass die Metallpartikel auch an der Oberfläche innerhalb des Elastomers elektrisch leitend bleiben und nicht zu einer nichtleitenden Schicht an der Oberfläche oxidieren.

Die Elektroden 26, 27, 28 sindauf der Innenoberseite des Bodies als leitfähige Schicht aufgebracht und haben die Gestalt einer Kreisscheibe mit einem Durchmesser von ca. 1,5 cm. Sie sind in der ähnlichen Weise aufgebaut, wie die Widerstandsschicht 48. Sie bestehen aus einem Elastomer 53, in das wiederum elektrisch leitende Partikel 52 eingebettet sind. Der Anschlussdraht 37 wird mit einem abisolierten Ende 54 in die noch nicht ausgehärtete elastomere Masse eingebettet und dadurch sowohl kontaktiert, als auch mechanisch festgelegt, wie dies auch bei den Dehnungsmessstreifen 29, 30 der Fall ist.

Die Oberfläche kann glatt oder strukturiert sein. Im Falle einer Strukturierung setzt sich die Oberfläche aus einer Anordnung von Tetraedern oder Pyramiden oder einem Textiloberflächenimitat zusammen, wodurch der Schweißtransport, der Tragekomfort und die Drapierbarkeit sowie der Übergangswiderstand verbessert werden. Die Elektrode kann auch vollständig als Textil ausgeführt sein, indem elektrisch leitfähige Garne oder Fäden zu einer textilen Fläche verarbeitet sind. Diese Fläche kann entweder in der vorgesehenen Form und Größe aufgenäht oder beim Stricken des Gürtels als Intarsie eingearbeitet werden.

Da es bei der Elektrode nicht auf eine Widerstandsänderung ankommt, sondern um einen möglichst geringen Widerstand, ist der Anteil von elektrisch leitenden Partikeln 52 gegebenenfalls höher (>50% Volumenanteil).

Anstelle der erwähnten Kohlepartikel können auch Metallpartikel verwendet werden. Bei der Auswahl des geeigneten Materials ist jedoch darauf zu achten, dass die Metallpartikel auch nach dem Aushärten des Polymers keine elektrisch isolierende Oxydschicht haben. Sie würden sonst lediglich als nichtleitende Füllkörper dienen, was den Zweck der Maßnahme vereiteln würde.

Bei dem Ausführungsbeispiel nach Fig. 1 war der Body 1 beispielsweise durch Rundstricken, anschließendes Zuschneiden und Besäumen der Kanten erzeugt worden. Die Verbindungskabel sind als separate Bänder produziert und anschließend aufgenäht.

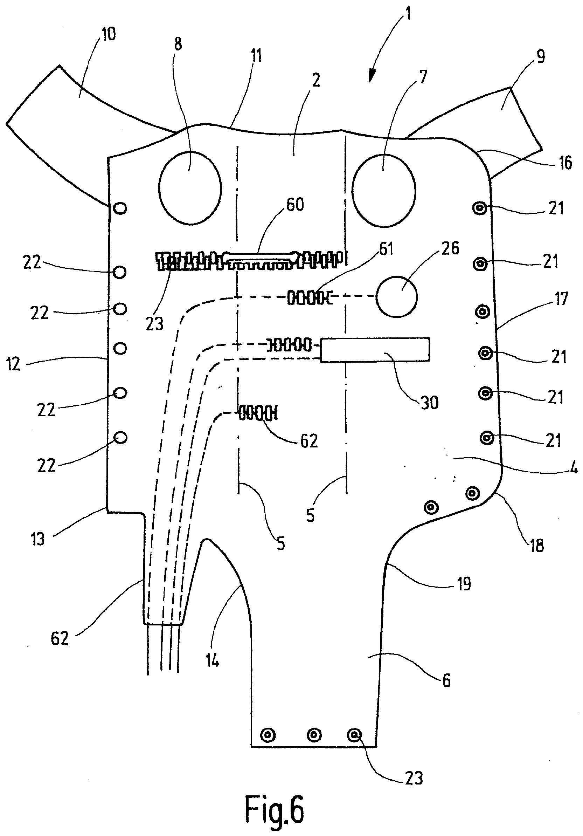

Fig. 6 zeigt eine Ausführungsform die nach dem so genannten "fully fashioned"-Verfahren hergestellt ist. Hierbei handelt es sich um ein spezielles Flachstrickverfahren, bei dem die gewünschte Struktur (mit Ausnahme der Ärmel 9 und 10) in einem Arbeitsgang in der entsprechend gewünschten Gestalt produziert wird.

Dabei wird eine unterschiedliche Dehnbarkeit im Rückenbereich 2 erhalten, indem dort, wie gezeigt, in dem Gestrick 23 einzelne Fäden 60 als Flottung liegen, d.h. nicht abgestrickt sind. Unter Flottung versteht der Fachmann, dass die Fäden dort in Richtung der Maschenreihe, ohne Ausbildung von Maschen liegen. Hierdurch wird die Dehnbarkeit wegen der fehlenden Maschenstruktur eingeschränkt.

Weiterhin ist es möglich, wie bei 61 gezeigt, leitfähige Fäden unmittelbar mit einzustricken, um die Kontaktierung des Sensors 26 zu erreichen. Die eingestrickten Fäden laufen zunächst in Richtung der Maschenreihe, d.h. sie bilden Maschenreihen, oder sie sind, zusammen mit dem Grundmaterial, als plattierte Fäden mit verstrickt. In der Nähe der Seitenkante 12 werden diese leitfähigen Fäden, die die Anschlussdrähte bilden dann in Richtung der Maschenstäbchen eingebunden, um an einer angestrickten Lasche 62 als freie Enden auszutreten, damit sie dort an einem Stecker entsprechend dem Stecker 42 kontaktiert werden können.

In ähnlicher Weise geschieht der Anschluss des Dehnungssensors 30, in dem dort im Abstand voneinander und somit elektrisch voneinander isoliert, mehrere Drähte mit eingestrickt werden, um die elektrische Kontaktierung zu erreichen.

Zweckmäßigerweise werden pro elektrische Leitung mehrere Leiter mit eingestrickt, um eine gewisse Redundanz zu erhalten, damit die elektrische Kontaktierung auch nicht verloren geht, sollte einer der Leiter brechen.

Damit Körperschweiß, der von dem textilen Grundmaterial aufgesogen wird, keinen unerwünschten Kurzschluss zwischen den Leitern herstellt, werden zweckmäßigerweise Drähte mit verstrickt, die für sich isoliert sind.

Schließlich können durch spezielle Mustertechniken, wie sie bei der Jaquardtechnik bekannt sind, Strukturen, wie bei 62 angedeutet, mit eingestrickt werden, um dort beispielsweise eine metallisch blanke Kontaktfläche zu erzeugen.

Der Vorteil der Herstellungstechnik des Bodys nach Fig. 1 besteht in den geringeren Anforderungen an die Komplexität der verwendeten Strick- und Webmaschinen. Dafür sind eine Reihe von Zuschnitt- und Näharbeiten erforderlich. Die Zuschnitt- und Näharbeiten werden bei der Ausführungsform nach Fig. 6 wesentlich reduziert. Dafür sind kompliziertere Textilmaschinen erforderlich.

Das Grundprinzip der Erfindung ist zuvor anhand eines Bodys erläutert. Dieser Body kann sehr wohl für Säuglinge, Kleinkinder als auch für Erwachsene verwendet werden. Der wesentliche Vorteil besteht darin, dass sowohl für im Bett liegende Patienten/Personen verwendet werden kann, als auch bei der normalen Tätigkeit oder bei Sport getragen werden kann.

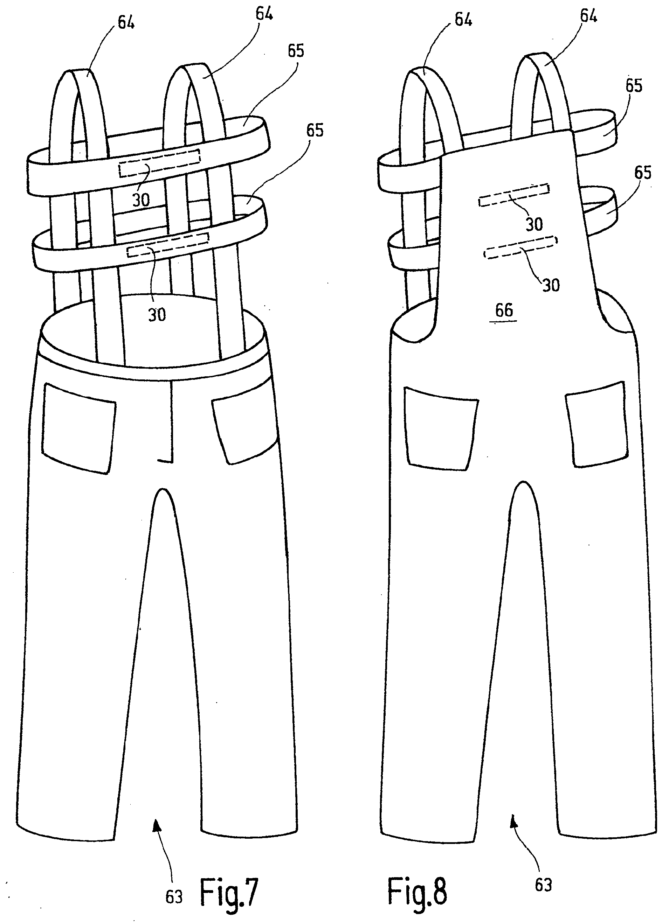

Eine andere Möglichkeit zur Umsetzung der Erfindung ist in den Fig. 7 und 8 gezeigt. Die Art des Kleidungsstücks, mit Hilfe dessen das Monitoring hindurchgeführt wird, beschränkt sich nicht auf Bodies. Vielmehr können auch Hosen 63 gemäß den Fig. 7 und 8 verwendet werden. Bei Fig. 7 wird die Hose mit Hilfe von Trägern 64 gehalten, die über Gürtel 65 miteinander verbunden sind. Die Gürtel 65 tragen gestrichelt angedeutete Sensoren 30 auf der dem Körper zugewandten Seite. Die Gürtel laufen wiederum in Querrichtung zur Körperlängsachse und liegen aufgrund ihrer Eigenelastizität am Körper an. Weitere Sensoren können ohne weiteres auf der dem Körper zugewandten Seite der Träger 64 angebracht werden. Aufgrund der Vorspannung der im Brustbereich verlaufenden Gürtels 65 werden die Träger ebenfalls eng anliegend an der Körperoberfläche gehalten, um Messungen vorzunehmen, wie sie im Zusammenhang mit Fig. 1 erläutert wurden.

Bei dem Ausführungsbeispiel nach Fig. 8 handelt es sich um eine Latzhose mit einem Latz 66, an dessen dem Körper zugewandten Seite die Sensoren 30 angebracht sind. Die Gürtel 65 gehen seitlich von dem Latz 66 aus und umschließen den Körper des Trägers. Sie drücken, wie erläutert, elastisch den Latz 66 mit den auf der Innenseite befindlichen Sensoren 30 gegen die Hautoberfläche. Außerdem gehen von der Oberkante des Latzes 66 Träger 64 aus, die zum Bund der Hose 63 führen.

Durch das Eigengewicht des unteren Teils der Hose 64 wird verhindert, dass beim Tragen die an den Trägern 64 bzw. den Gürteln 65 angebrachten Sensoren in unerwünschter Weise nach oben wandern und den vorgeschriebenen Messort am Körper verlassen.

Das Kleidungsstück, wie es in den Fig. 7 und 8 gezeigt ist, eignet sich insbesondere auch zur Überwachung der Körperfunktionen bei Menschen, die einer normalen Tätigkeit nachgehen und die volle Beweglichkeit benötigen.

Gemäß Fig. 1 wird zum Anschließen der Sensoren 29, 30 das Bandkabel 38 verwendet, das als flaches Band mit geschlossenen Rändern gewebt ist. Etwa auf der Höhe des Abzweigs 34 ist das Bandkabel der Länge nach eingeschnitten, um durch Umschlagen die F-förmige Gestalt zu bekommen.

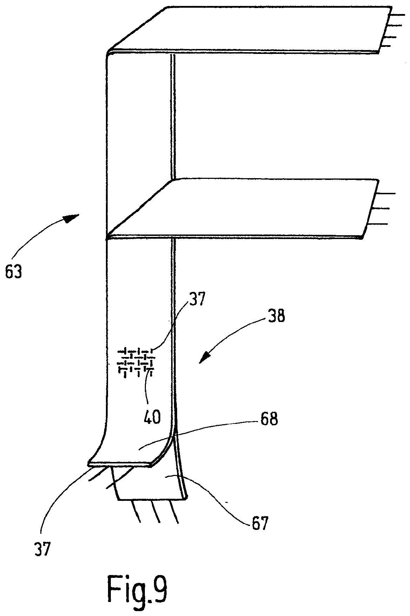

Wenn sehr viele Elektroden oder Sensoren angeschlossen werden müssen, ist es unter Umständen schwierig, die vielen Drähte in einer Ebene, wie sie ein einfaches flaches Band ergibt, als Kettfäden unterzubringen. Für eine sehr große Anzahl von Verbindungsleitungen oder -drähten eignet sich insbesondere die Struktur nach Fig. 9. Hierbei besteht das Anschlusskabel 38 aus einem gewebten Schlauch. Ein solcher gewebter Schlauch ist in Umfangsrichtung endlos und bildet gedanklich zwei Bänder 67 und 68, die längs ihrer beiden Ränder durch den schraubenförmig verlaufenden Schussfaden einstückig miteinander verbunden sind. Hierdurch wird ein zweilagiges Gebilde geschaffen, wobei in jeder Lage Anschlussdrähte 37 untergebracht werden können. Die Anschlussdrähte verlaufen wiederum in Kettrichtung.

Auf der gewünschten Höhe bei 69 werden die beiden Lagen 67 und 68 voneinander getrennt und, wie gezeigt, umgeklappt, um die gewünschte F-förmige Struktur zu bekommen.

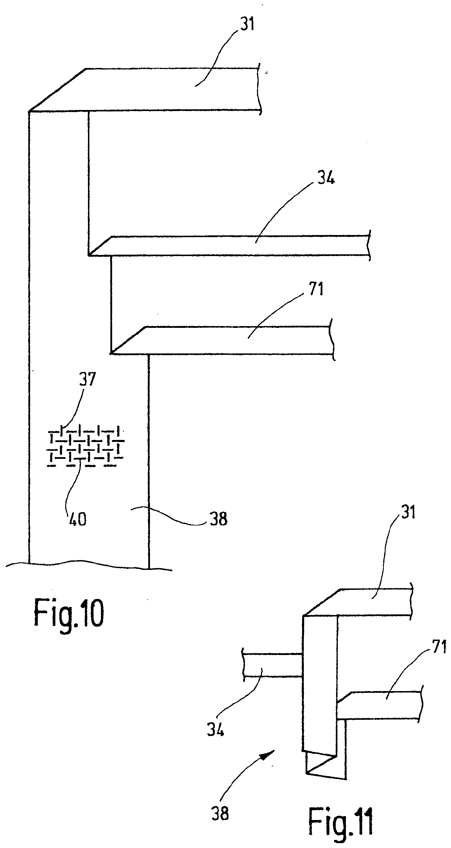

Fig. 10 lässt erkennen, dass mit Hilfe des bandförmigen Kabels 38 nicht nur zwei Abgänge 31 und 34, sondern mehr Abgänge, beispielsweise drei Abgänge 31, 34, 71 möglich sind. Hierzu wird das Band nach dem Weben in der gewünschten Weise in Längsrichtung parallel zu den Kettfäden getrennt und umgeschlagen.

Gemäß Fig. 11 ist das verhältnismäßig breite Band 10, dessen Gesamtbreite im flachliegenden Zustand so breit ist, wie die Summe der Breiten der einzelnen Abzweigleitungen 31, 34, 71, leporelloartig gefaltet. Dadurch reduziert sich die Breite des bandförmigen Kabels 38 auf die Breite des breitesten Abzweigs, beispielsweise des Abzweigs 31. Außerdem kann ein "Kabelbaum" erzeugt werden, bei dem die einzelnen Abzweigleitungen 31, 34, 71 zu unterschiedlichen Seiten weg führen. Ein Wegführen zur gleichen Seite, d.h. ein F mit drei Armen ist ohne weiteres ebenfalls zu erzeugen.

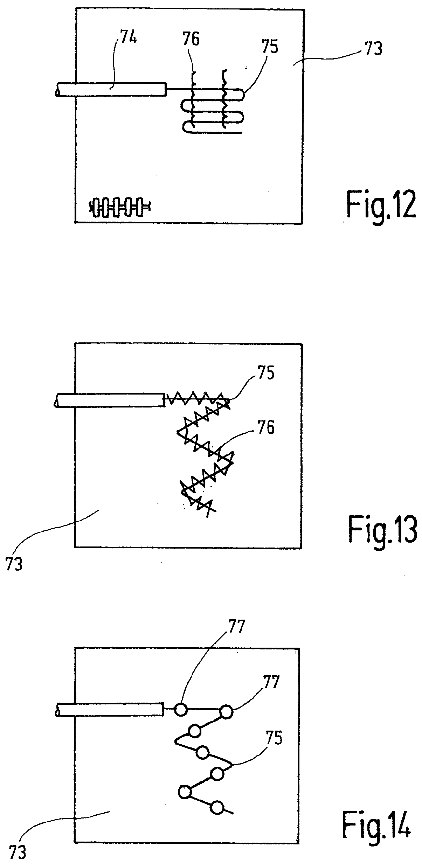

In den Fig. 12 bis 17 sind eine Reihe von Verfahren veranschaulicht, wie der Leiter eines isolierten Drahtes mit einer textilen Unterlage 73 verbindbar ist. Ein isolierter Leiter 74 ist ein Stück weit abisoliert, so dass der im Inneren des Leiters 74 enthaltene Draht 75 blank liegt. Mit Hilfe von Nähfäden 76 ist der blanke Teil des Drahtes auf das elektrisch nicht leitende testile Substrat 73 aufgenäht.

Gemäß Fig. 13 wird der abisolierte Draht 75 mit Hilfe eines Fadens 76 auf der Unterlage festgestickt.

Bei dem Ausführungsbeispiel nach Fig. 14 ist der blank liegende Draht 75 mit Hilfe von Klebepunkten 77 befestigt. Anstelle von separaten Klebepunkten 77 kann, wenn das textile Substrat schmelzklebefähige Fäden enthält, der abisolierte Draht 75 auch durch Aufschmelzen dieser Fäden bis in den Klebzustand auf dem Substrat befestigt werden. Das Aufschmelzen kann durch Wärme oder mittels Ultraschall erzielt werden.

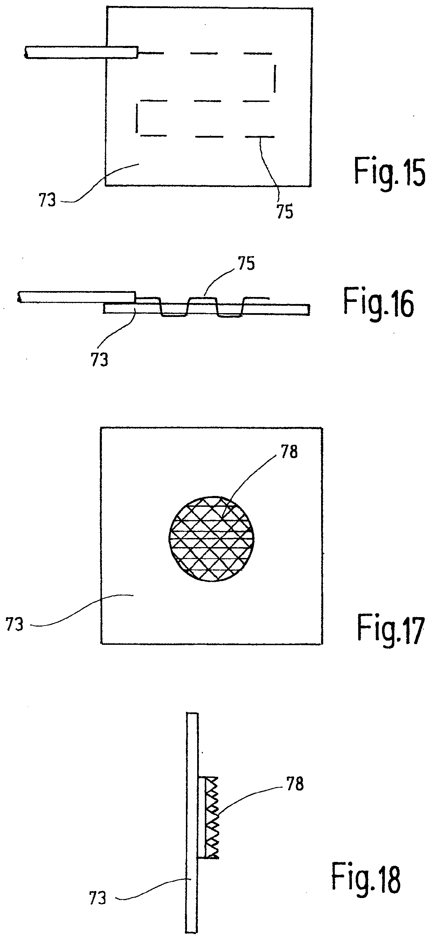

Die Fig. 15 und 16 veranschaulichen, wie der abisolierte Draht 75 als Faden in das Substrat 73 eingenäht wird. Wie Fig. 16 erkennen lässt, erscheint der Draht 75 dabei abwechselnd auf den beiden Seiten des textilen Substrates. Als textiles Substrat kommen Gewebe, Maschenware oder Vlies in Frage.

Die zuvor erwähnten Sensoren aus Elastomer liegen flächig auf der Haut auf und dichten diesen Teil der Haut weitgehend ab. Hautausdünstungen können nur schwer unter dem Sensor austreten. Um die Belüftung und das Ablaufen von Schweiß zu verbessern, kann die Sensoroberfläche, wie in Fig. 17 gezeigt, strukturiert sein. Sie setzt sich beispielsweise aus einer Vielzahl kleiner Pyramiden 78 zusammen, deren Spitzen der Haut zugekehrt sind. Bei mäßigem Andruck entstehen zwischen den Spitzen Kanälchen, durch die hindurch Schweiß ablaufen kann.

Unterhalb der gezeigte Fläche, kann der zum Kontaktieren verwendete Draht 75 gemäß den Fig. 12-16 angebracht sein, oder mit Hilfe einer Jaquardstricktechnik, wie dies anhand von Fig. 6 erläutert ist.

Der Dehnungssensor nach Fig. 2 besteht aus einem Elastomer, das mit elektrisch leitenden Partikeln gefüllt ist. Als dehnungsabhängiger Sensor können aber auch Hydrogele eingesetzt werden. Ein solcher Sensor enthält ein Hydrogel, das mit einer Elektrolytlösung gefüllt ist. Hierbei wird in einer dreidimensional vernetzen Matrix aus hydrophilen wasserunlöslichen Polymeren Wasser eingelagert und dadurch quasi immobilisiert. Geeignete Hydrogele sind Polymetacrylate, Polyphenylpyrolidone oder Polyphenylalkohol. Dem in der Hydrogelschicht gespeicherten Wasser wird ein wasserlösliches Salz zugegeben, um eine Ionenleitfähigkeit des Wassers zu erreichen. Als Salz eignet sich AgCl-Salz sowie jedes andere physiologisch unbedenkliche Metallsalz, beispielsweise Kochsalz. Eine Querschnittsänderung zufolge einer Längenänderung durch Dehnung oder durch Druck beeinflusst die Leitfähigkeit. Der gemessene Widerstand ist damit ein Maß für die Dehnung, der der mit einem Hydrogel ausgerüsteter Sensor ausgesetzt ist.

Das Hydrogel befindet sich quasi als Füllung zwischen zwei wasser- und ionendichten hochelastischen Schichten, ähnlich wie dies für die leitfähige Schicht 48 in Fig. 3 gezeigt ist. Somit entspricht der Aufbau eines auf einem Hydrogel basierenden Sensors dem Aufbau, wie er in Fig. 3 dargestellt ist, wobei anstelle des leitfähigen Elastomers 48 das Hydrogel eingesetzt ist. Als Elastomer kommt Silicon in Frage.

Der Vorteil von Hydrogelen besteht darin, dass, abhängig vom Vernetzungsgrad, eine sehr weiche und damit bequem am Körper zu tragende Beschaffenheit erreicht werden kann.

Das erfindungsgemäße Kleidungsstück ist in Verbindung mit einem Body im Einzelnen erläutert. der Body stellt die bevorzugte Ausführungsform dar. Es ist jedoch auch möglich, die gezeigten Sensoren an Westen oder Unterhemden zu befestigen soweit diese Kleidungsstücke eng anliegend am Körper getragen werden.

In einem Body verlaufen quer zur Längsachse des Trägers ein oder zwei Gürtel, die in Längsrichtung dehnbar sind. In diesen Gürteln sind Dehnungsmessstreifen untergebracht. An der Außenseite der Gürtel, die mit dem Körper Kontakt macht, befinden sich Elektroden zum Ableiten von Herzströmen oder zum Messen des Hautwiderstands.

## Patentansprüche

1. Kleidungsstück, das zumindest abschnittsweise in zumindest einer Richtung dehnbar ist und dessen Konstruktion/Schnitt derart gestaltet ist, dass es im angelegten Zustand am menschlichen Körper lagerichtig fixiert bleibt,
mit wenigstens einem Sensor (25,26,27,28,29,30) zum Erfassen wenigstens einer Körperfunktion wie Hautwiderstand, Atmung, Puls, Herzströme, Körpertemperatur, Transpiration und Ähnlichem, wobei der Sensor (25,26,27,28,29,30) an dem Kleidungsstück (1) mittels eines zumindest abschnittsweise dehnbaren Gürtel (31,34) befestigt ist und zur Abgabe eines elektrisches Signals eingerichtet ist,
mit wenigstens einem weiteren Gürtel (31,34) zum Anschluss wenigstens eines weiteren Sensors (25,26,27,28,29,30), und
mit einem Verbindungskabel (38), das in dem Kleidungsstück (1) befestigt ist, um die elektrische Verbindung zu dem Sensor (25,26,27,28,29,30) herzustellen,
wobei die Gürtel (31,34) und das Verbindungskabel (38) eine F-förmige Konfiguration bilden, und
wobei zur Kontaktierung der Sensoren (25,26,27,28,29,30) isolierte Einzeladern (37) vorgesehen sind, die als Fäden in dem Gürtel (31,34) verlaufen,
**dadurch gekennzeichnet, dass** das Verbindungskabel (38) ein zweilagiges Gebilde mit zwei Lagen (67, 68) ist, die zur Bildung der F-förmigen Konfiguration voneinander getrennt und umgeklappt sind, oder dass das Verbindungskabel (38) ein Bandkabel ist, das zur Bildung der F-förmigen Konfiguration der Länge nach getrennt und umgeschlagen ist.

2. Kleidungsstück nach Anspruch 1, **dadurch gekennzeichnet, dass** es sich um einen so genannten Body handelt, der Thorax und Abdomen umhüllt und mit einem Halsausschnitt, zwei Armausschnitten (7,8) und zwei Beinausschnitten versehen ist.

3. Kleidungsstück nach Anspruch 1, **dadurch gekennzeichnet, dass** der Body (1) in Längsrichtung, vorzugsweise auf der Vorderseite, zu öffnen ist.

4. Kleidungsstück nach Anspruch 1, **dadurch gekennzeichnet, dass** der Body (1) wenigstens einen vorzugsweise angeschnittenen Schrittspickel (6) enthält, die durch den Schritt geführt wird.

5. Kleidungsstück nach Anspruch 1, **dadurch gekennzeichnet, dass** der Body (1) mit Ärmeln (9,10) versehen ist.

6. Kleidungsstück nach Anspruch 1, **dadurch gekennzeichnet, dass** das Kleidungsstück (1) eine Weste ist.

7. Kleidungsstück nach Anspruch 1, **dadurch gekennzeichnet, dass** das Kleidungsstück (1) nach Art eines T-Shirt ausgebildet ist.

8. Kleidungsstück nach Anspruch 1, **dadurch gekennzeichnet, dass** Kleidungsstück (1) nach Art eines Unterhemds mit Trägern ausgeführt ist.

9. Kleidungsstück nach Anspruch 1, **dadurch gekennzeichnet, dass** Kleidungsstück (1) nach Art einer Hose (63) mit Trägern und/oder Hosenlatz (64,66) ausgeführt ist, wobei Querbänder (65) enthalten sind.

10. Kleidungsstück nach Anspruch 1, **dadurch gekennzeichnet, dass** es sich bei dem Material ein textiles Flächengebilde handelt.

11. Kleidungsstück nach Anspruch 10, **dadurch gekennzeichnet, dass** es sich bei dem textilen Flächengebilde um eine Maschenware (23) handelt.

12. Kleidungsstück nach Anspruch 1, **dadurch gekennzeichnet, dass** der Sensor (29,30) ein Sensor ist, der bei Streckung seinen Widerstandswert verändert.

13. Kleidungsstück nach Anspruch 11, **dadurch gekennzeichnet, dass** der Sensor (29,30) einen spezifischen Widerstandswert von ca. 25 Ohm x cm aufweist.

14. Kleidungsstück nach Anspruch 1, **dadurch gekennzeichnet, dass** der Sensor (26,27,28) ein flexibler Sensor ist, dessen Widerstandswert im Wesentlichen konstant ist.

15. Kleidungsstück nach Anspruch 12 oder 14, **dadurch gekennzeichnet, dass** der Sensor (25,26,27,28,29,30) ein elastomeres Material (53) als Grundmaterial aufweist, in dem leitfähige Partikel (52) eingebettet sind.

16. Kleidungsstück nach Anspruch 12 oder 14, **dadurch gekennzeichnet, dass** es sich bei den leitfähigen Partikeln (52) um Kohlepartikel oder leitfähigen Metallpartikeln handelt, jeweils mit einem Durchmesser zwischen 0,01 µm und 10 µm, wobei das Material der Metalle aus den Stoffen Al, Cu, Ag, Fe, Ni, Ti, Legierungen mit diesen Metallen ausgewählt ist und jeder Zwischenwert, der zwischen den oben genannten Grenzen liegt als neuer Grenzwert mit beansprucht ist.

17. Kleidungsstück nach Anspruch 12 oder 14, **dadurch gekennzeichnet, dass** der Volumenanteil insbesondere der Kohlepartikel zwischen 30% und 60% liegt, wobei jeder Zwischenwert, der zwischen den oben genannten Grenzen liegt als neuer Grenzwert mit beansprucht ist.

18. Kleidungsstück nach Anspruch 15, **dadurch gekennzeichnet, dass** das Elastomer (53) ein hautverträgliches Elastomer ist.

19. Kleidungsstück nach Anspruch 15, **dadurch gekennzeichnet, dass** das Elastomer (53) nicht allergen ist.

20. Kleidungsstück nach Anspruch 12 oder 14, **dadurch gekennzeichnet, dass** das Elastomer (53) eine stärkere Dehnbarkeit aufweist, als das Substrat (31,34) auf den der Sensor (29,30) angebracht ist.

21. Kleidungsstück nach Anspruch 15, **dadurch gekennzeichnet, dass** das Elastomer (53) aus den Stoffen Fluorpolymer, Polyurethan, Silicon ausgewählt ist.

22. Kleidungsstück nach Anspruch 12 oder 14, **dadurch gekennzeichnet, dass** der Sensor (29,30) auf lediglich einer Seite mit einer Isolierschicht (51) versehen ist.

23. Kleidungsstück nach Anspruch 12, **dadurch gekennzeichnet, dass** der Sensor (29,30) allseitig von einer Isolierschicht (47,51) umgeben ist.

24. Kleidungsstück nach Anspruch 21 oder 22, **dadurch gekennzeichnet, dass** die isolierende Schicht (47,51) oder isolierenden Schichten des Sensors (29,30) aus demselben Material bestehen wie die aktive Schicht (47), jedoch keine leitfähige Füllung (52) enthalten.

25. Kleidungsstück nach Anspruch 12 oder 14, **dadurch gekennzeichnet, dass** der Sensor (25,26,27,28,29,30) über wenigstens einen Anschlussdraht (37) kontaktiert ist.

26. Kleidungsstück nach Anspruch 12 oder 14, **dadurch gekennzeichnet, dass** der Sensor (29,30) die Gestalt einer vorzugsweise flachen Bahn hat, deren Quererstreckung klein ist gegenüber der Längserstreckung.

27. Kleidungsstück nach Anspruch 25, **dadurch gekennzeichnet, dass** die Bahn des Sensors (29,30) U-förmig verläuft und das die Kontaktierung an den Enden der Bahn (44,46) erfolgt.

28. Kleidungsstück nach Anspruch 14, **dadurch gekennzeichnet, dass** der Sensor (25,26,27,28) scheibenförmig/flächig ist und eine runde oder eckige Form aufweist.

29. Kleidungsstück nach Anspruch 12 oder 14, **dadurch gekennzeichnet, dass** der Sensor (25,26,27,28,29,30) aus einem Material besteht, das unempfindlich gegen Körperschweiß ist.

30. Kleidungsstück nach Anspruch 12 oder 14, **dadurch gekennzeichnet, dass** der Sensor (25,26,27,28,29,30) aus einem Material besteht, das unempfindlich gegen übliche Textilpflege- und/oder Waschmittel ist.

31. Kleidungsstück nach Anspruch 12 oder 14, **dadurch gekennzeichnet, dass** der Sensor (25,26,27,28,29,30) aus Material besteht, das warmwasserfest ist.

32. Kleidungsstück nach Anspruch 12 oder 14, **dadurch gekennzeichnet, dass** der Sensor (25,26,27,28,29,30) aus Material besteht, das heißwasserfest ist.

33. Kleidungsstück nach Anspruch 12 oder 14, **dadurch gekennzeichnet, dass** der Sensor (25,26,27,28,29,30) aus Material besteht, das eine Sterilisation, insbesondere in einem Autoklaven, übersteht.

34. Kleidungsstück nach Anspruch 12 oder 14, **dadurch gekennzeichnet, dass** der Sensor ein Hydrogel enthält.

35. Kleidungsstück nach Anspruch 12 oder 14, **dadurch gekennzeichnet, dass** der Sensor eine strukturierte Oberfläche aufweist.

36. Kleidungsstück nach Anspruch 1, **dadurch gekennzeichnet, dass** sich der Gürtel (31,34) zumindest abschnittsweise auf der Innenseite des Kleidungsstücks (1) befindet.

37. Kleidungsstück nach Anspruch 1, **dadurch gekennzeichnet, dass** der Gürtel (31,34) von einem flach liegenden Schlauch gebildet ist, in dem der Sensor (29,30) untergebracht ist.

38. Kleidungsstück nach Anspruch 1, **dadurch gekennzeichnet, dass** der Gürtel (31,34) von einem flach liegenden Schlauch gebildet ist, auf/in dem der Sensor (25,26,27,28,29,30) untergebracht ist.

39. Kleidungsstück nach Anspruch 1, **dadurch gekennzeichnet, dass** der Gürtel (31,34) aus einer Maschenware besteht.

40. Kleidungsstück nach Anspruch 1, **dadurch gekennzeichnet, dass** der Gürtel (31,34) in dem Kleidungsstück (1) quer zur Längsachse des Körpers verläuft.

41. Kleidungsstück nach Anspruch 1, **dadurch gekennzeichnet, dass** in dem Kleidungsstück (1) die Gürtel (31,34) parallel zueinander verlaufen.

42. Kleidungsstück nach Anspruch 1, **dadurch gekennzeichnet, dass** der Gürtel (31,34) mit einem Längsabschnitt in dem Kleidungsstück (1) fest eingenäht ist und dass ein anderer Abschnitt frei ist, wobei das freie Ende dieses Abschnittes mit einer Verankerungseinrichtung (33,36) in Form eines Druckknopfes, eines Klettbandes oder dergleichen versehen ist.

43. Kleidungsstück nach Anspruch 1, **dadurch gekennzeichnet, dass** der eine Gürtel (31) im Brustbereich knapp unterhalb der Armausschnitte (7,8) des Kleidungsstücks (1) verläuft.

44. Kleidungsstück nach Anspruch 36, **dadurch gekennzeichnet, dass** der zweite Gürtel (34) im Bereich des Bauches verläuft um eine Bauchatmung zu erfassen.

45. Kleidungsstück nach Anspruch 36, **dadurch gekennzeichnet, dass** der festgenähte Abschnitt des Gürtels (31,34) in einen in dem Kleidungsstück (1) ausgebildeten Schlauch (41) einmündet, der in Längsrichtung der Körperachse verläuft.

46. Kleidungsstück nach Anspruch 1, **dadurch gekennzeichnet, dass** die isolierten Einzeladern (37) die Kettfäden in dem Gewebe (38) bilden.

47. Kleidungsstück nach Anspruch 1, **dadurch gekennzeichnet, dass** die isolierten Einzeladern (37) unmittelbar als Stehfäden eine Wirkware eingearbeitet sind.

48. Gürtel oder Kleidungsstück nach Anspruch 1, **dadurch gekennzeichnet, dass** elektrische Leiter unmittelbar als Fäden in das textile Grundmaterial eingearbeitet sind.

49. Gürtel oder Kleidungsstück nach Anspruch 1, **dadurch gekennzeichnet, dass** die Einzeladern (37) direkt in die textile Fläche als Stehfäden oder als Flottung liegende Fäden eingearbeitet sind.

50. Kleidungsstück nach Anspruch 1, **dadurch gekennzeichnet, dass** es im fully fashioned Verfahren hergestellt ist.

## Claims

1. Article of clothing which at least in portions can be stretched in at least one direction, and the construction/cut of which is formed such that in the state applied to the human body, it remains fixed in the correct position,
with at least one sensor (25, 26, 27, 28, 29, 30) for detecting at least one bodily function such as skin resistance, respiration, pulse, cardiac currents, body temperature, perspiration and similar, wherein the sensor (25, 26, 27, 28, 29, 30) is attached to the article of clothing (1) by means of a belt (31, 34) which is stretchable at least in portions and configured to emit an electrical signal,
with at least one further belt (31, 34) for connection of at least one further sensor (25, 26, 27, 28, 29, 30), and
with a connecting cable (38) which is secured in the article of clothing (1) in order to create the electrical connection to the sensor (25, 26, 27, 28, 29, 30),
wherein the belt (31, 34) and the connecting cable (38) form an F-shaped configuration, and
wherein isolated individual cores (37) running as threads in the belt (31, 34) are provided for contacting the sensors (25, 26, 27, 28, 29, 30),
**characterised in that** the connecting cable (38) is a two-layered structure with two layers (67, 68) which are separated from each other and folded to form an F-shaped configuration, or that the connecting cable (38) is a ribbon cable which is separated and folded longitudinally to form the F-shaped configuration.

2. Article of clothing according to claim 1, **characterised in that** it is a so-called body which envelops the thorax and abdomen and is provided with a neck opening, two arm openings (7, 8) and two leg openings.

3. Article of clothing according to claim 1, **characterised in that** the body (1) is opened in the longitudinal direction, preferably on the front.

4. Article of clothing according to claim 1, **characterised in that** the body (1) contains at least one preferably cut crotch piece (6) which is guided through the crotch.

5. Article of clothing according to claim 1, **characterised in that** the body (1) is provided with sleeves (9, 10).

6. Article of clothing according to claim 1, **characterised in that** the article of clothing (1) is a waistcoat.

7. Article of clothing according to claim 1, **characterised in that** the article of clothing (1) is formed in the manner of a T-shirt.

8. Article of clothing according to claim 1, **characterised in that** the article of clothing (1) is formed in the manner of a singlet with shoulder straps.

9. Article of clothing according to claim 1, **characterised in that** the article of clothing (1) is formed in the manner of trousers (63) with braces and/or flies (64, 66), wherein cross belts (65) are included.

10. Article of clothing according to claim 1 , **characterised in that** the material is a flat textile structure.

11. Article of clothing according to claim 10, **characterised in that** the flat textile structure is a knitted fabric (23).

12. Article of clothing according to claim 1, **characterised in that** the sensor (29, 30) is a sensor which changes its resistance value when stretched.

13. Article of clothing according to claim 11, **characterised in that** the sensor (29, 30) has a specific resistance value of around 25 Ohm x cm.

14. Article of clothing according to claim 1, **characterised in that** the sensor (26, 27, 28) is a flexible sensor, the resistance value of which is substantially constant.

15. Article of clothing according to claim 12 or 14, **characterised in that** the base material of the sensor (25, 26, 27, 28, 29, 30) is an elastomer material (53) in which conductive particles (52) are embedded.

16. Article of clothing according to claim 12 or 14, **characterised in that** the conductive particles (52) are carbon particles or conductive metal particles, each with a diameter of between 0.01 µm and 10 µm, wherein the material of the metals is selected from the substances Al, Cu, Ag, Fe, Ni, Ti, alloys with these metals, and each intermediate value lying between the above-mentioned limits is also claimed as a new limit value.

17. Article of clothing according to claim 12 or 14, **characterised in that** the volume proportion in particular of the carbon particles is between 30% and 60%, wherein each intermediate value lying between the above-mentioned limits is also claimed as a new limit value.

18. Article of clothing according to claim 15, **characterised in that** the elastomer (53) is an elastomer which can be tolerated by the skin.

19. Article of clothing according to claim 15, **characterised in that** the elastomer (53) is not allergenic.

20. Article of clothing according to claim 12 or 14, **characterised in that** the elastomer (53) has a greater stretchability than the substrate (31, 34) on which the sensor (29, 30) is attached.

21. Article of clothing according to claim 15, **characterised in that** the elastomer (53) is selected from the substances fluoropolymer, polyurethane, silicon.

22. Article of clothing according to claim 12 or 14, **characterised in that** the sensor (29, 30) has an isolating layer (51) on one side only.

23. Article of clothing according to claim 12, **characterised in that** the sensor (29, 30) is surrounded by an isolating layer (47, 51) on all sides.

24. Article of clothing according to claim 21 or 22, **characterised in that** the isolating layer (47, 51) or isolating layers of the sensor (29, 30) consist of the same material as the active layer (47) but without a conductive filling (52).

25. Article of clothing according to claim 12 or 14, **characterised in that** the sensor (25, 26, 27, 28, 29, 30) is contacted via at least one connecting wire (37).

26. Article of clothing according to claim 12 or 14, **characterised in that** the sensor (29, 30) has the form of a preferably flat track, the transverse extension of which is small in comparison with the longitudinal extension.

27. Article of clothing according to claim 25, **characterised in that** the track of the sensor (29, 30) runs in a U-shape, and the contacting takes place at the ends of the track (44, 46).

28. Article of clothing according to claim 14, **characterised in that** the sensor (25, 26, 27, 28) is disc-shaped/flat and has a round or angular form.

29. Article of clothing according to claim 12 or 14, **characterised in that** the sensor (25, 26, 27, 28, 29, 30) consists of a material which is not sensitive to body perspiration.

30. Article of clothing according to claim 12 or 14, **characterised in that** the sensor (25, 26, 27, 28, 29, 30) consists of a material which is not sensitive to conventional textile care and/or washing agents.

31. Article of clothing according to claim 12 or 14, **characterised in that** the sensor (25, 26, 27, 28, 29, 30) consists of a material which is resistant to warm water.

32. Article of clothing according to claim 12 or 14, **characterised in that** the sensor (25, 26, 27, 28, 29, 30) consists of a material which is resistant to hot water.

33. Article of clothing according to claim 12 or 14, **characterised in that** the sensor (25, 26, 27, 28, 29, 30) consists of a material which survives sterilisation, in particular in an autoclave.

34. Article of clothing according to claim 12 or 14, **characterised in that** the sensor contains a hydrogel.

35. Article of clothing according to claim 12 or 14, **characterised in that** the sensor has a structured surface.

36. Article of clothing according to claim 1, **characterised in that** the belt (31, 34) is situated at least in portions on the inside of the article of clothing (1).

37. Article of clothing according to claim 1, **characterised in that** the belt (31, 34) is formed by a flat lying hose in which the sensor (29, 30) is accommodated.

38. Article of clothing according to claim 1, **characterised in that** the belt (31, 34) is formed by a flat lying hose, in or on which the sensor (25, 26, 27, 28, 29, 30) is accommodated.

39. Article of clothing according to claim 1, **characterised in that** the belt (31, 34) consists of a knitted fabric.

40. Article of clothing according to claim 1, **characterised in that** the belt (31, 34) in the article of clothing (1) runs transversely to the longitudinal axis of the body.

41. Article of clothing according to claim 1, **characterised in that** in the article of clothing (1), the belts (31, 34) run parallel to each other.

42. Article of clothing according to claim 1, **characterised in that** the belt (31, 34) is fixedly stitched by a longitudinal portion to the article of clothing (1), and that another portion is free, wherein the free end of this portion is provided with an anchoring device (33, 36) in the form of a press stud, a hook and loop tape, or similar.

43. Article of clothing according to claim 1, **characterised in that** the one belt (31, 34) runs in the chest region just below the arm openings (7, 8) of the article of clothing (1).

44. Article of clothing according to claim 36, **characterised in that** the second belt (31, 34) runs in the region of the abdomen in order to detect abdominal respiration.

45. Article of clothing according to claim 36, **characterised in that** the fixedly stitched portion of the belt (31, 34) opens into a hose (41) formed in the article of clothing (1) and running in the longitudinal direction of the body axis.

46. Article of clothing according to claim 1, **characterised in that** the isolated individual cores (37) form the warp threads of the woven fabric (38).

47. Article of clothing according to claim 1, **characterised in that** the isolated individual cores (37) are worked directly into a knitted material as filler threads.

48. Belt or article of clothing according to claim 1, **characterised in that** electrical conductors are worked directly into the textile base material as threads.

49. Belt or article of clothing according to claim 1, **characterised in that** the individual cores (37) are worked directly into the textile material as filler threads or as threads lying as floats.

50. Article of clothing according to claim 1, **characterised in that** it is produced in the fully fashioned process.

## Revendications

1. Article d'habillement qui, au moins localement, est extensible dans au moins une direction et dont la construction/coupe est configurée de telle manière qu'à l'état porté il reste de façon fixe en place sur le corps humain,
comportant au moins un capteur (25, 26, 27, 28, 29, 30) destiné à capter au moins une fonction corporelle telle que la résistance de la peau, la respiration, le pouls, les flux cardiaques, la température du corps, la transpiration et des fonctions analogues, le capteur (25, 26, 27, 28, 29, 30) étant fixé sur l'article d'habillement (1) au moyen d'une ceinture (31, 34) extensible au moins localement et qui est agencé en vue de délivrer un signal électrique,
comportant au moins une autre ceinture (31, 34) destinée au raccordement d'au moins un autre capteur (25, 26, 27, 28, 29, 30),
et comportant un câble de liaison (38) qui est fixé dans l'article d'habillement (1) en vue de réaliser une liaison électrique avec le capteur (25, 26, 27, 28, 29, 30),
les ceintures (31, 34) et le câble de liaison (38) formant une configuration en forme de F,
des conducteurs individuels isolés (37) étant prévus, lesquels s'étendant en tant que fils dans la ceinture (31, 34),
**caractérisé en ce que** le câble de liaison (38) a une structure à deux couches comportant deux couches (67, 68) qui sont séparés l'une de l'autre en vue de réaliser la configuration en forme de F et qui sont repliées, ou **en ce que** le câble de liaison (38) est un câble du type ruban qui, en vue de la formation de la configuration en forme de F, est divisé dans le sens de la longueur et replié.

2. Article d'habillement selon la revendication 1 **caractérisé en ce qu'**il s'agit de ce que l'on appelle un body qui enveloppe le thorax et l'abdomen et qui est doté d'une ouverture pour le cou, de deux ouvertures (7, 8) pour les bras et de deux ouvertures pour les jambes.

3. Article d'habillement selon la revendication 1 **caractérisé en ce que** le body (1) doit être ouvert dans la direction de la longueur, de préférence sur le devant.

4. Article d'habillement selon la revendication 1 **caractérisé en ce que** le body (1) comporte au moins une patte d'entrejambe (6) qui a été de préférence découpée et qui passe dans l'entrejambe.

5. Article d'habillement selon la revendication 1 **caractérisé en ce que** le body (1) est muni de manches (9, 10).

6. Article d'habillement selon la revendication 1 **caractérisé en ce que** l'article d'habillement (1) est une veste.

7. Article d'habillement selon la revendication 1 **caractérisé en ce que** l'article d'habillement (1) est conformé à la manière d'un T-shirt.

8. Article d'habillement selon la revendication 1 **caractérisé en ce que** l'article d'habillement (1) est exécuté à la manière d'un sous-vêtement avec bretelles.

9. Article d'habillement selon la revendication 1 **caractérisé en ce que** l'article d'habillement (1) est exécuté à la manière d'un pantalon (63) avec bretelles et/ou avec baguette (64, 66), des bandes transversales (65) étant présentes.

10. Article d'habillement selon la revendication 1 **caractérisé en ce qu'**il s'agit, en ce qui concerne le matériau, d'un textile structuré.

11. Article d'habillement selon la revendication 10 **caractérisé en ce qu'**il s'agit d'un tricot (23) en ce qui concerne le textile structuré.

12. Article d'habillement selon la revendication 1 **caractérisé en ce que** le capteur (29, 30) est un capteur qui en cas d'allongement modifie sa valeur de résistance.

13. Article d'habillement selon la revendication 11 **caractérisé en ce que** le capteur (29, 30) présente une valeur spécifique de résistance d'environ 25 Ohm x cm.

14. Article d'habillement selon la revendication 1 **caractérisé en ce que** le capteur (26, 27, 28) est un capteur souple dont la valeur de résistance est essentiellement constante.

15. Article d'habillement selon la revendication 12 ou 14 **caractérisé en ce que** le capteur (25, 26, 27, 28, 29, 30) présente, en tant que matériau de base, un matériau élastomère (53) dans lequel sont incorporées des particules conductrices (52).

16. Article d'habillement selon la revendication 12 ou 14 **caractérisé en ce qu'**il s'agit, en ce qui concerne les particules conductrices (52) de particules de carbone ou de particules métalliques, conductrices dans tous les cas d'un diamètre compris entre 0,01 micromètres et 10 micromètres, le matériau des métaux étant choisis parmi les éléments qui suivant : Al, Cu, Ag, Fe, Ni, Ti et des alliages de ces métaux et **en ce que** chaque valeur intermédiaire qui se situe entre les limites citées plus haut étant revendiquée comme nouvelle valeur limite.

17. Article d'habillement selon la revendication 12 ou 14 **caractérisé en ce que** la proportion en volume, en particulier celle des particules de carbone se situe entre 30 % et 60 %, chaque valeur intermédiaire qui se situe entre les limites citées plus haut étant revendiquée comme nouvelle valeur limite.

18. Article d'habillement selon la revendication 15 **caractérisé en ce que** l'élastomère (53) est un élastomère supportable par la peau.

19. Article d'habillement selon la revendication 15 **caractérisé en ce que** l'élastomère (53) n'est pas allergénique.

20. Article d'habillement selon la revendication 12 ou 14 **caractérisé en ce que** l'élastomère (53) présente une extensibilité plus forte que le substrat (31, 34) sur lequel le capteur (29, 30) est disposé.

21. Article d'habillement selon la revendication 15 **caractérisé en ce que** l'élastomère (53) est choisi parmi les élastomères du type polymère fluoré, du type polyuréthane, du type silicone.

22. Article d'habillement selon la revendication 12 ou 14 **caractérisé en ce que** le capteur (29, 30) est muni uniquement sur un côté d'une couche isolante (51).

23. Article d'habillement selon la revendication 12 **caractérisé en ce que** le capteur (29, 30) est entouré de tous côtés d'une couche isolante (47, 51).

24. Article d'habillement selon la revendication 21 ou 22 **caractérisé en ce que** la couche isolante (47, 51), ou les couches isolantes du capteur (29, 30) est constituée/sont constituées du même matériau que la couche active (47) mais ne contient/ne contiennent aucune charge conductrice (52).

25. Article d'habillement selon la revendication 12 ou 14 **caractérisé en ce que** le capteur (25, 26, 27, 28, 29, 30) est mis en contact par l'intermédiaire d'au moins un fil de raccordement (37).

26. Article d'habillement selon la revendication 12 ou 14 **caractérisé en ce que** le capteur (29, 30) a de préférence la forme d'une bande plate dont l'étendue dans le sens transversal est faible par rapport à son étendue dans le sens longitudinal.

27. Article d'habillement selon la revendication 25 **caractérisé en ce que** la bande du capteur (29, 30) s'étend en forme de U et **en ce que** la mise en contact s'effectue aux extrémités de la bande (44, 46).

28. Article d'habillement selon la revendication 14 **caractérisé en ce que** le capteur (25, 26, 27, 28) a une forme de disque plat et présente une forme ronde, ou comportant des angles.

29. Article d'habillement selon la revendication 12 ou 14 **caractérisé en ce que** le capteur (25, 26, 27, 28, 29, 30) est constitué d'un matériau qui est insensible à la sueur corporelle.

30. Article d'habillement selon la revendication 12 ou 14 **caractérisé en ce que** le capteur (25, 26, 27, 28, 29, 30) est constitué d'un matériau qui est insensible aux soins habituels apportés aux textiles et/ou aux produits de lavage.

31. Article d'habillement selon la revendication 12 ou 14 **caractérisé en ce que** le capteur (25, 26, 27, 28, 29, 30) est constitué d'un matériau qui résiste à l'eau chaude.

32. Article d'habillement selon la revendication 12 ou 14 **caractérisé en ce que** le capteur (25, 26, 27, 28, 29, 30) est constitué d'un matériau qui résiste à l'eau très chaude.

33. Article d'habillement selon la revendication 12 ou 14 **caractérisé en ce que** le capteur (25, 26, 27, 28, 29, 30) est constitué d'un matériau qui résiste à une stérilisation, en particulier dans un autoclave.

34. Article d'habillement selon la revendication 12 ou 14 **caractérisé en ce que** le capteur contient un gel hydrique.

35. Article d'habillement selon la revendication 12 ou 14 **caractérisé en ce que** le capteur présente une surface structurée.

36. Article d'habillement selon la revendication 1 **caractérisé en ce que** la ceinture (31, 34) se trouve au moins localement sur le côté intérieur de l'article d'habillement (1).

37. Article d'habillement selon la revendication 1 **caractérisé en ce que** la ceinture (31, 34) est formée d'un fourreau disposé à plat dans lequel est logé le capteur (29, 30).

38. Article d'habillement selon la revendication 1 **caractérisé en ce que** la ceinture (31, 34) est formée d'un fourreau disposé à plat sur lequel/dans lequel est logé le capteur (25, 26, 27, 28, 29, 30).

39. Article d'habillement selon la revendication 1 **caractérisé en ce que** la ceinture (31, 34) est constituée d'un tricot.

40. Article d'habillement selon la revendication 1 **caractérisé en ce que** la ceinture (31, 34) s'étend dans l'article d'habillement (1) transversalement à l'axe longitudinal du corps.

41. Article d'habillement selon la revendication 1 **caractérisé en ce que** les ceintures (31, 34) s'étendent parallèlement l'une à l'autre dans l'article d'habillement (1).

42. Article d'habillement selon la revendication 1 **caractérisé en ce que** la ceinture (31, 34) est cousue par un segment longitudinal qui est cousu solidement dans l'article d'habillement (1) et **en ce qu'**un autre segment est libre, l'extrémité libre de ce segment étant munie d'un dispositif d'ancrage (33, 36) se présentant sous la forme d'un bouton-pression, d'un ruban agrippant ou d'un moyen semblable.

43. Article d'habillement selon la revendication 1 **caractérisé en ce que** l'une des ceintures (31) s'étend dans la zone de la poitrine juste en-dessous des emmanchures (7, 8) de l'article d'habillement (1).

44. Article d'habillement selon la revendication 36 **caractérisé en ce que** la deuxième ceinture (34) s'étend dans la zone du ventre en vue de capter la respiration abdominale.

45. Article d'habillement selon la revendication 36 **caractérisé en ce que** le segment de la ceinture (31, 34) cousu solidement débouche dans un fourreau (41) formé dans l'article d'habillement (1), lequel fourreau s'étend dans le sens longitudinal de l'axe corporel.

46. Article d'habillement selon la revendication 1 **caractérisé en ce que** les conducteurs individuels (37) isolés forment des fils de chaîne dans le tissage (38).

47. Article d'habillement selon la revendication 1 **caractérisé en ce que** les conducteurs individuels (37) sont incorporés directement en tant que fils de remplissage d'un tricot.

48. Ceinture ou article d'habillement selon la revendication 1 **caractérisé en ce que** des conducteurs électriques individuels (37) sont incorporés directement en tant que fils dans le matériau textile de base.

49. Ceinture ou article d'habillement selon la revendication 1 **caractérisé en ce que** les conducteurs individuels (37) sont incorporés directement dans la surface textile en tant que fils de remplissage ou en tant que fils flottants.

50. Article d'habillement selon la revendication 1 **caractérisé en ce qu'**il est fabriqué selon le procédé dit fully fashioned.
